# EUROPEAN PATENT SPECIFICATION

(11) **EP 0 971 722 B1**
(45) Date of publication and mention of the grant of the patent: **07.05.2003**
(21) Application number: 97904999.6
(22) Date of filing: 27.02.1997
(51) Int. Cl.: A61K 35/78, A61P 15/00

(54) **AN APHRODISIAC FOR APPLICATION TO THE SEXUAL ORGANS**
APHRODISIAKUM ZUR ANWENDUNG AN DEN GESCHLECHTSORGANEN
APHRODISIAQUE DESTINE A UNE APPLICATION SUR LES ORGANES SEXUELS

(30) Priority: 11.03.1996 DK 27996
(43) Date of publication of application: 19.01.2000
(73) Proprietor: Erozir Danmark ApS, 9440 Aabybro (DK)
(72) Inventor: Bjeldbak, Gitte, 9510 Arden (DK)
(74) Representative: Larsen, Hans Ole
(86) International application number: PCT/DK97/00085
(87) International publication number: WO 97/033597

(56) References cited:
- US-A- 4 795 638
- US-A- 5 384 123
- DRUG & AGING, Volume 7, No. 4, 1995, CHRIS RAINS et al., "Topical Capsaicin. A Review of Its Pharmacological Properties and Therapeutic Potential in Post-Herpetic Neuralgia, Diabetic Neuropathy and Osteoarthritis", pages 317-328.

## Description

The present invention is concerned with a natural product having a sexually stimulating action, intended to be applied in diluted form to a woman's or man's sexual organs.

Among the known agents to be found on the market I have not found any that is rubbed directly on the sexual organs that has the action that my invention has.

The agent has a sexually stimulating action within 1-120 seconds after application.

The sexual stimulation is achieved by diluting chilli pepper and rubbing it directly on the sexual organs. The proportions of chilli pepper and diluent, which may be e.g. an oil, cream, or any lubricant, are dependent on whether fresh chilli or chilli powder is used.

## Claims

1. Use of pure chilli or an oil, cream or lubricant comprising pure chilli in fresh or powdered dry or dried form in the preparation of an aphrodisiac for application to the sexual organs of a man or a woman.

## Patentansprüche

1. Verwendung von reinem Chili oder einem Öl, einer Creme oder einem Gleitmittel enthaltend reinen Chili in frischer oder pulverisierter trockener oder getrockneter Form bei der Zubereitung eines Aphrodisiakums zur Anwendung an den Geschlechtsorganen eines Mannes oder einer Frau.

## Revendications

1. Utilisation de piment pur ou d'une huile, d'une crème ou d'un lubrifiant comprenant du piment pur frais, sec ou sous forme de poudre pour la préparation d'un aphrodisiaque destiné à être appliqué sur les organes sexuels d'un homme ou d'une femme.
